# EUROPEAN PATENT SPECIFICATION

(11) **EP 2 550 985 B1**
(45) Date of publication and mention of the grant of the patent: **09.09.2020**
(21) Application number: 12005389.7
(22) Date of filing: 24.07.2012
(51) Int. Cl.: A61M 5/315

(54) **Plunger kit for medicine syringe**
Stößelsatz für Medizinspritze
Kit de piston pour seringue médicale

(30) Priority: 25.07.2011 JP 2011162321
(43) Date of publication of application: 30.01.2013
(73) Proprietor: Daikyo Seiko, LTD., Sano-shi, Tochigi 327-0813 (JP)
(72) Inventor: Sudo, Nobuo, Tochigi 327-0813 (JP)
(74) Representative: Blodig, Wolfgang

(56) References cited:
- EP-A1- 2 407 195
- EP-A1- 2 484 393
- JP-A- 2007 289 677
- JP-A- 2009 165 524
- US-B1- 6 481 760

## Description

### Field of the Invention

This invention relates to a plunger kit for medicine syringe (which may hereinafter be called simply "plunger kit for syringe" or "plunger kit"), as a component of a syringe for use with a medicine, and specifically to a plunger kit composed of a piston and a plunger rod threadedly engageable with each other. When the plunger rod is brought into threaded engagement with the piston, the plunger rod is resistant to coming out of the piston without any substantial increase in the sliding resistance of the piston in a syringe barrel, and the plunger kit is suited especially for a small-capacity syringe. In addition, this invention is also concerned with a medicine syringe including a syringe barrel and the above-described plunger kit slidably fitted in a threadedly-engaged form in the syringe barrel.

### Background of the Invention

A medicine syringe is used to inject a medical solution into the human body or the like, and upon its use, is required to perform a drawing operation of the medical solution from a storage container into the syringe and an ejection operation for the administration of the drawn medical solution. A prefilled syringe which has been finding increased utility in recent years has been filled with a medical solution in the syringe when it is sold from a pharmaceutical maker. The prefilled syringe, therefore, does not require the above-described drawing operation of the medical solution, but a drawing operation called "nurse aspiration" is needed upon use. As a consequence, it is required for the prefilled syringe to be equipped with a new function to hold the medical solution in a sealed state within the syringe until use in addition to the drawing and ejecting functions required for conventional syringes. Such sealing performance is generally assured by a nozzle cap and a piston. The nozzle cap serves to seal a nozzle as a needle hub of the syringe, and the piston acts to seal the syringe on the side of its basal end.

Plunger kits employed in such syringes include a variety of types. Roughly classifying, there are the integrated type and the separate type. In a plunger kit of the integrated type, a piston and a plunger rod for operating the piston are inseparably integrated together. In a plunger kit of the separate type, on the other hand, a piston and a plunger rod are provided with an internal screw and an external screw, respectively, and upon use of an associated syringe, they are brought into threaded engagement and are then used. The separate type is advantageous in that it is not voluminous during storage and shipping. However, the separate type relies upon the threaded engagement for the connection between the piston and the plunger rod and, as the piston is made of soft rubber in general, involves a problem in that the piston and plunger rod may disengage from each other in a sudden during a drawing operation.

To solve such a problem, the applicant has already proposed a plunger kit for syringe (see U.S. Patent No. 6, 129, 712) . In the plunger kit, a piston is provided with an internal screw, which is designed small in the width of its basic groove profile and large in the width of its inter-groove thread portion, and a plunger rod is provided with an external screw, which is designed large in the width of its basic thread profile and small in the width of its inter-thread groove portion. This plunger kit does not have the potential problem that the plunger rod may separate from the piston when the plunger rod is pulled, and has brought advantageous results.

From the standpoint of reducing a burden on patients, there is a move toward smaller dose sizes in recent years. Keeping in step with this, small-capacity syringes are now finding ever-increasing utility. For such a small-capacity syringe, the above-mentioned invention described in U.S. Patent No. 6,129,712 is a very effective technology provided that it is of such a size as permitting the designing of a threaded portion of its plunger rod as desired. If the threaded portion is of a small shape, however, the above-described invention can be hardly applied in view of the moldability of the threaded portion. As the capacity of a syringe has become extremely small especially as in recent years and its plunger kit has become smaller correspondingly, it has become more difficult to precisely perform the molding of screws, leading to more cases where the provision of a plunger kit with sufficient thread engagement strength is unavoidably difficult. For increased thread engagement strength, for example, it may be contemplated to design the external screw of a plunger rod a little greater compared with the internal screw of a piston. In this case, however, the external screw expands the piston together with the internal screw so that the outer diameter of the piston becomes greater. As a result, the sliding resistance of the piston in a syringe barrel increases, thereby causing another problem that the sliding performance of the piston deteriorates.
EP 2407195 A1 and EP 2484393 A1, which are prior art according to Art. 54(3) EPC, disclose gaskets and syringes.
JP 2009 165524 A discloses a plunger for a syringe.
US 6481760 B discloses a connection for two metal pipes.
JP 2007 289677 A discloses a plunger rod and gasket connection.

### Disclosure of the Invention

Therefore, an object of the present invention is to solve these problems and to provide a plunger kit, which includes a piston and plunger rod that constitute a syringe for use with a medicine and which can be integrated by bringing the piston and plunger rod into threaded engagement upon use, specifically a novel plunger kit that with a plunger rod being in threaded engagement with a piston, the plunger rod is resistant to coming out of the piston without any substantial increase in the sliding resistance of the piston even when a syringe is small in capacity and shape.

The above-described object can be achieved by the present invention to be described hereinafter. Described specifically, the present invention provides the subject matter of claims 1 to 11, in particular, a plunger kit as a component for a medicine syringe provided with a syringe barrel and useful for injecting a medicine, said plunger kit comprising a piston made of an elastomer and having an internal screw and a plunger rod made of a material harder than the piston and having an external screw, characterized in that the internal screw and external screw have a mutually non-complementary, basic groove profile and basic thread profile, respectively; and a following flank of each thread of the external screw and a groove flank of a corresponding groove of the internal screw are formed such that, when the piston and plunger rod are brought into threaded engagement with each other and slidably fit in the syringe barrel and the piston is then slid in a pull direction by way of the plunger rod, the following flank can come into contact with the groove flank, which is located face-to-face with the following flank, more strongly at a first part of the following flank than at a second part of the following flank and more weakly at the second part than at the first part to increase thread engagement strength between the external screw and the internal screw without any substantial expansion of the piston by the external screw. Even when the piston is slid in the pull direction by way of the plunger rod in the syringe barrel with the plunger rod being in threaded engagement with the piston, the plunger rod is resistant to coming out of the piston without any substantial increase in the sliding resistance of the piston even when the syringe is small in capacity and shape.

The angle of the following flank with respect to the pull direction of the piston and the angle of the groove flank with respect to the push direction of the piston are each from 90 to 150°, and the angles are different from each other. As an example, one of the basic groove profile of the internal screw and the basic thread profile of the external screw may have a trapezoidal screw thread form, and the other may have one of a round thread form, a square thread form and another trapezoidal screw form having sloping sides inclinations of which are different from those of sloping sides of the first-mentioned trapezoidal screw form having sloping sides inclinations of which are different from those of sloping sides of the first-mentioned trapezoidal screw thread form. As another example, one of the following flank and the groove flank, which is located face-to-face with the following flank, may have a flat surface, and the other may have a curved surface. The curved surface may be formed preferably on at least a part of the following flank. As a further example, one of the basic groove profile of the internal screw and the basic thread profile of the external screw may have a round thread form, and the other may have a trapezoidal screw thread form or a square thread form.

In the above-described plunger kit, a leading flank of each thread of the external screw and an opposite groove flank of the corresponding groove of the internal screw can be formed such that, when the piston is slid in a push direction by way of the plunger rod in the syringe barrel, the leading flank can come into contact with the opposite groove flank, which is located face-to-face with the leading flank, more strongly at a third part of the leading flank than at a fourth part of the leading flank and more weakly at the fourth part than at the third part to increase thread engagement strength between the external screw and the internal screw without any substantial expansion of the piston by the external screw. This plunger kit is also useful when sufficient thread engagement strength is desired to remain between the external screw even when the piston is slid in the push direction by way of the plunger rod.

The external screw and internal screw may preferably be set in profile and dimensions such that each two adjacent threads of the external screw can come into contact, at root-side portions thereof, with an inter-groove thread portion of the internal screw, said inter-groove thread portion being located between the two threads, with the inter-groove thread portion being clamped at free end portions thereof by the root-side portions, to produce a clamping load between the internal screw and the external screw. As an alterative, each thread of the external screw and its corresponding groove of the internal screw may preferably be set in profile and dimensions such that the thread can come into contact, at end corner portions thereof located between a crest thereof and a leading flank and following flank thereof, respectively, with groove flanks of the groove, said groove flanks being located face-to-face with the leading flank and following flank, respectively, to produce a clamping load between the internal screw and the external screw. As another alternative, each thread of the external screw may preferably be provided, on intermediate parts of a leading flank and following flank thereof, with shoulder portions, respectively, and the thread of the external screw and the corresponding groove of the internal screw may preferably be set in profile and dimensions such that the shoulder portions can come into contact with the groove flanks of the groove, respectively, to produce a clamping load between the internal screw and the external screw.

Preferably, the piston may have the internal screw threadedly engageable with the plunger rod, the internal screw may define therein a cylinder space and grooves (in other words, a screw groove) helically extending from the cylinder space toward an outer circumference of the piston, the plunger rod may have the external screw threadedly engageable with the piston, and the external screw may have a cylinder and threads (in other words, a screw thread) helically extending in a radial and outward direction from the cylinder.

The present invention also provides, in another aspect thereof, a medicine syringe characterized by comprising a syringe barrel and the above-described plunger kit slidably fitted in a threadedly-engaged form in the syringe barrel.

### Brief Description of the Drawings

FIG. 1A is an enlarged schematic view of a cross-section of a free end section of a plunger kit according to related art.
FIG. 1B is an enlarged fragmentary schematic view for describing a threadedly engaged state of threaded portions of FIG. 1A.
FIG. 2A is an overall schematic view illustrating a plunger kit according to a first embodiment of the present invention in which a piston is in a disengaged state.
FIG. 2B is an enlarged schematic view of a cross-section of a free end section of the plunger kit of FIG. 2A in which the piston is in an engaged state.
FIG. 2C is an enlarged fragmentary schematic view of FIG. 2B for describing thread portions in an engaged state.
FIG. 2D is a schematic view for describing changes at the threaded portions in FIG. 2C, which occur upon sliding of the piston.
FIG. 3 is an enlarged schematic view of threaded portions for describing a plunger kit according to a second embodiment of the present invention.
FIG. 4A is an enlarged schematic view of threaded portion for describing a plunger kit according to a third embodiment of the present invention.
FIG. 4B is an enlarged schematic view of threaded portions for describing a plunger kit according to a fourth embodiment of the present invention.
FIG. 5A is an enlarged schematic view of threaded portions for describing a plunger kit according to a fifth embodiment of the present invention.
FIG. 5B is an enlarged schematic view of threaded portions for describing a plunger kit according to a sixth embodiment of the present invention.
FIG. 5C is a schematic view for describing changes at the threaded portions in FIG. 5B, which occur upon sliding of a piston.

### Detailed Description of Preferred Embodiments

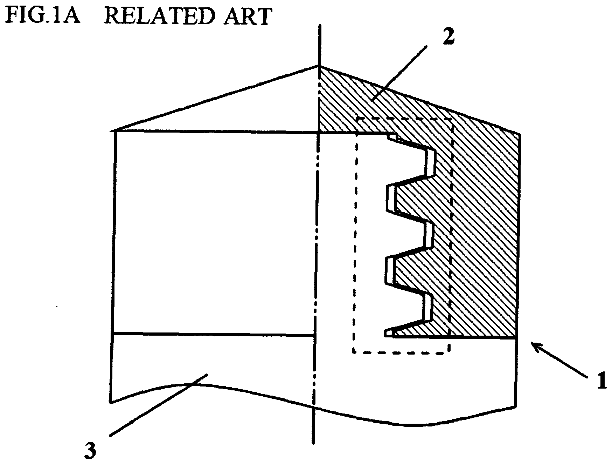

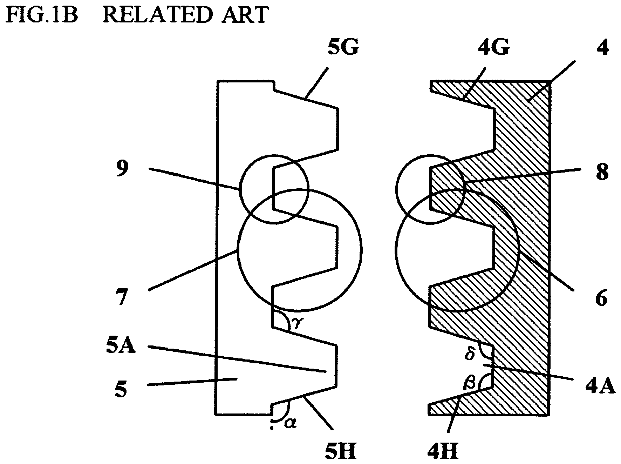

With reference to the drawings illustrating best modes for practicing the present invention, the present invention will next be described in further detail. FIG. 1A is an enlarged fragmentary schematic view of a cross-section of a free end section of a conventional plunger kit 1 according to related art when an internal screw of a piston 2 and an external screw of a plunger rod 3 have been brought into threaded engagement with each other. In FIG. 1B, there is shown an enlarged fragmentary schematic view, in which to describe the profiles of threaded portions in the related art and the basic construction of the internal screw 4 and external screw 5 of mutually-engageable structures, a portion surrounded by broken lines in FIG. 1A has been enlarged and is illustrated by separating the surrounded portion into an internal screw portion and an external screw portion. Referring to these figures, a description will hereinafter be made about the basic structures of the internal screw 4 and external screw 5, and further, about the threaded engagement between the internal screw 4 and external screw 5. Definitions will first be given on certain terms used in the present invention.

The term "basic groove profile" as used in the present invention shall mean the profile of each groove as a basis for defining the profile of the groove to be brought into threaded engagement with a thread. As designated at numeral 6 in FIG. 1B, the basic groove profile shall indicate the cross-sectional profile of a single groove. The term "basic thread profile", on the other hand, shall mean the profile of each thread as a basis for defining the profile of the thread. As designated at numeral 7 in FIG. 1B, the basic groove profile shall indicate the cross-sectional profile of a single thread. Further, a thread portion formed between each two adjacent grooves in the internal screw shall be called an "inter-groove thread portion" 8, and similarly, a groove portion formed between each two adjacent threads in the external screw shall be called an "inter-thread groove portion" 9. It is to be noted that the term "cross-section" as used in the present invention shall mean a cross-section that extends on and along a central axis of a piston or plunger rod and in parallel with a sliding direction of the piston.

Furthermore, the term "leading flank" of each thread of an external screw as used in the present invention shall mean a side wall of the thread, which is facing in a push direction when a piston is pushed by way of a plunger rod threadedly engaged with the piston in a syringe barrel. On the other hand, the term "following flank" of each thread shall mean a side wall on a side opposite to the leading flank. In other words, the term "following flank" of each thread shall mean a side wall of the thread, which is facing in a pull direction when the piston is pulled by way of the plunger rod threadedly engaged with the piston in the syringe barrel. Accordingly, the push direction of the piston and the pull direction of the piston are similar in meaning with a sliding direction of the piston in the syringe barrel, but are different from the sliding direction in that the directions are specified. In this specification, the expression "sliding direction" may be used simply without specifying in which one of the pull direction and push direction the piston is slid.

In addition, the expression "neither an angle of the following flank (or the leading flank) with respect to the pull (or push) direction of the piston nor an angle of the groove flank, which is located face-to-face with the following flank (or the leading flank), with respect to a push (or pull) direction of the piston is 180°" shall mean that a following flank 5H (or a leading flank 5G) of each thread 5A and a groove flank 4H(or 4G) of a corresponding groove 4A, said groove flank 4H(or 4G) being located face-to-face with the following flank 5H (or the leading flank 5G), as illustrated in FIG. 1B extends in a sliding direction of the piston in the syringe barrel, specifically "is not a wall parallel to a plane that is parallel to the sliding direction" which extends in a direction conforming to the up-and-down direction of the piston 2 illustrated in FIG. 1A as seen on the figure. In other words, the above-described expression means that concerning the walls specified in the present invention, the angles α,β,γ,δ shown in FIG. 1B are not 180° (α, β, γ, δ ≠ 180°) . In the present invention, the walls parallel to the sliding direction (in FIG. 1B, the crest of each thread 5A and the root of each groove 4A) are excluded from the elements or features that specify the present invention, because these parallel walls take substantially no part in the thread engagement strength between the external screw 5 and the. internal screw 4 during sliding of the piston. From the viewpoint of the above-described thread engagement strength, the above-mentioned angles may each be set at preferably from 90 to 150°, more preferably from 90 to 135°, most preferably from 90 to 130°.

In the above-described related art, the basic groove profile 6 of the internal screw 4 arranged on an inner wall of the piston 2 and the basic thread profile 7 of the external screw 5 arranged on an outer wall of a free end section of the plunger rod 3 are substantially complementary (α=β,γ=δ) as illustrated in FIGS. 1A and 1B. Such profiles can provide a greatest area of contact between the internal screw 4 and the external screw 5 upon threaded engagement, and therefore, have been considered to be preferable as a combination of profiles for screws.

The present inventor, however, found that such a combination of profiles is not absolutely preferable where the material of one of screws to be combined is soft and that of the other is hard as in a plunger kit for syringe. As a result of a great deal of research performed based on the above-described finding, the present inventor has completed the present invention. Described specifically, by making the basic groove profile of an internal screw of a piston and the basic thread profile of an external screw of a plunger rod non-complementary with each other and also by forming a following flank of each thread of the external screw and a groove flank of a corresponding groove of the internal screw such that, when the piston and plunger rod are brought into threaded engagement with each other and slidably fit in a syringe barrel and the piston is then slid in a pull direction by way of the plunger rod, the following flank can come into contact with the groove flank, which is located face-to-face with the following flank, more strongly at a first part of the following flank than at a second part of the following flank and more weakly at the second part than at the first part, the present inventor has made it possible to increase the thread engagement strength between the external screw and the internal screw without any substantial expansion of the internal screw and piston by the external screw when the piston is slid in the pull direction. By forming a leading flank of each thread of the external screw and an opposite groove flank of the corresponding groove of the internal screw such that, when the piston is slid in a push direction by way of the plunger rod in the syringe barrel, the leading flank can come into contact with the opposite groove flank, which is located face-to-face with the leading flank, more strongly at a third part of the leading flank than at a fourth part of the leading flank and more weakly at the fourth part than at the third part, the present inventor has also made it possible to increase the thread engagement strength without any substantial expansion of the internal thread and piston by the external screw when the piston is slid in the push direction. It is to be noted that, when the piston is slid in the push direction, strong thread engagement strength such as that required upon sliding the piston in the pull direction is not usually required in many instances. In such a case, the leading flank of each thread of the external screw and the groove flank of the corresponding groove of the internal thread, said groove flank being located face-to-face with the leading flank, may be formed as in the above-described related art, because these leading flank and the groove flank take part in the thread engagement strength only when the piston is slid in the push direction.

The thread engagement strength can be further increased by forming the internal screw and external screw in such profiles that a load can be produced between them at the stage of threaded engagement, in other words, in a state that no external load is applied on the plunger rod.

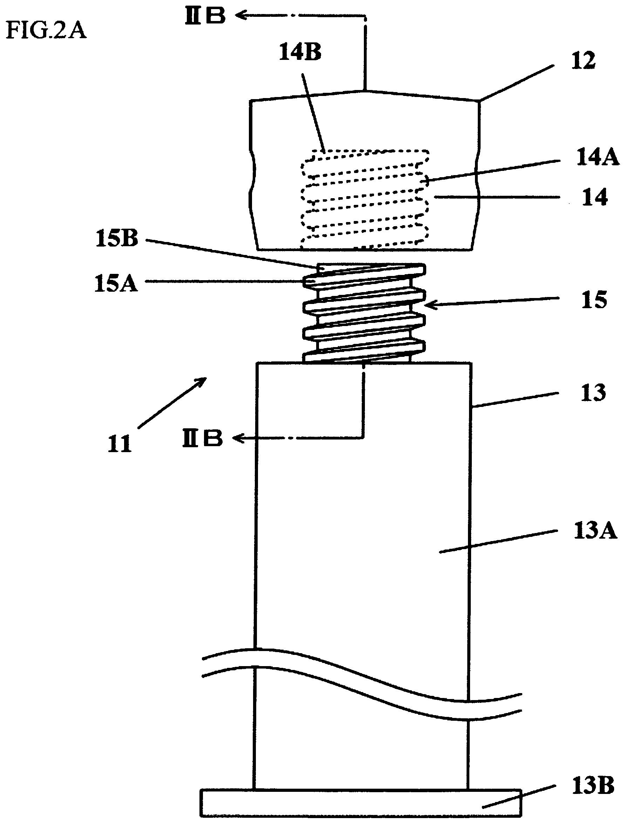

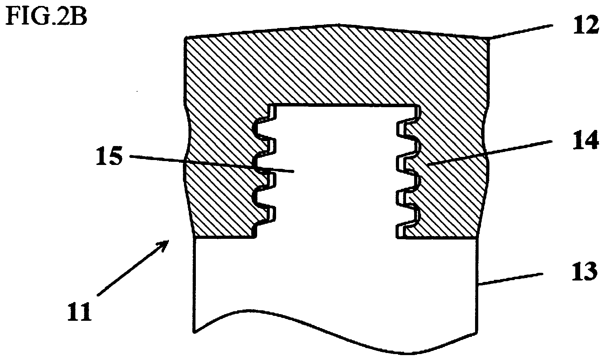

Referring to FIGS. 2A through FIG. 2D, a plunger kit according to a first embodiment of the present invention will be described. FIG. 2A is an overall schematic view of the plunger kit 11 according to the first embodiment, and illustrates a state in which a piston 12 is not in threaded engagement with a plunger rod 13. FIG. 2B is an enlarged schematic view of a cross-section of a free end section of the plunger kit 11 with the piston 12 being threadedly engaged with the plunger rod 13 as seen in the direction of arrows IIB-IIB in FIG. 2A.

As illustrated in FIG. 2A, the plunger kit 11 is composed of the piston 12 and plunger rod 13. The piston 12 is made of an elastomer such as soft rubber or thermoplastic elastomer, and is equipped with a function as a seal component for performing drawing and ejection of a medical solution by sliding, in an unillustrated syringe barrel, in an axial direction of the syringe barrel. This piston 12 generally has a substantially-cylindrical shape, and those having various outer circumferential shapes are known to achieve both sliding performance and sealing performance, including those having one or more ring-shaped grooves or ridges formed on their outer circumferences and those having an outer circumferential diameter increased or decreased depending on the location. The piston 12 illustrated in FIGS. 2A and 2B is an illustrative cylindrical piston with the diameter of its outer circumference being increased or decreased depending on the location. An internal screw 14 is bored through a bottom wall of a cylinder as the piston 12, in other words, a wall of the cylinder on a side where the piston 12 comes into contact with the plunger rod 13, and is arranged coaxially with the central axis of the cylinder. Also in FIG. 2A, the internal screw 14 is centrally defined by a cylinder space 14B and is also defined by grooves (in other words, a screw groove) 14A helically extending from the cylinder space 14B toward the outer circumference of the piston 12. There are some internal screws in each of which the boundary between a cylinder space and grooves (in other words, a screw groove) may not look clear. They, however, simply look so due to their groove profiles. Such internal screws shall, therefore, be included in the present invention.

As also depicted in FIG. 2A, the plunger rod 13 has a substantially-cylindrical, plunger-rod main body 13A, a flange 13B formed on a bottom wall of the main body 13A and employed as a finger rest upon operation of a syringe, and an external screw 15 formed on a top wall of the main body 13A and threadedly engageable with the internal screw 14 of the piston. As in the piston 12, the external screw 15 is formed coaxially with the central axis of the plunger rod main body 13A. Also as in the piston 12, the external screw 15 is centrally formed of a cylinder 15B and is also formed of threads (in other words, a screw thread) helically extending in a radial and outward direction from the cylinder 15B. The threaded engagement of the plunger rod 13 with the piston 12 results in such a state as illustrated in FIG. 2B.

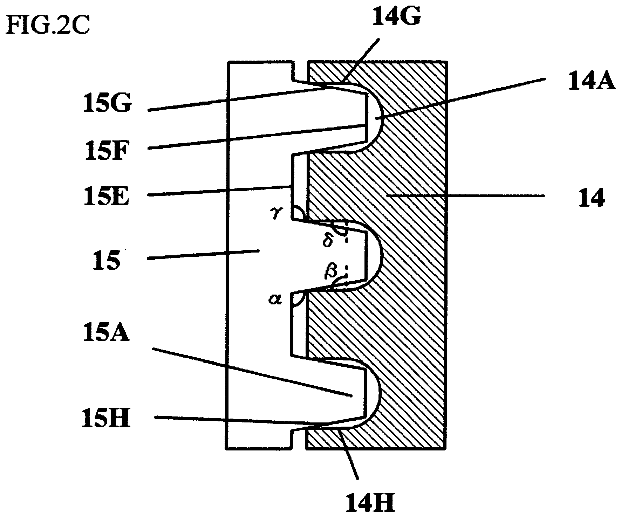

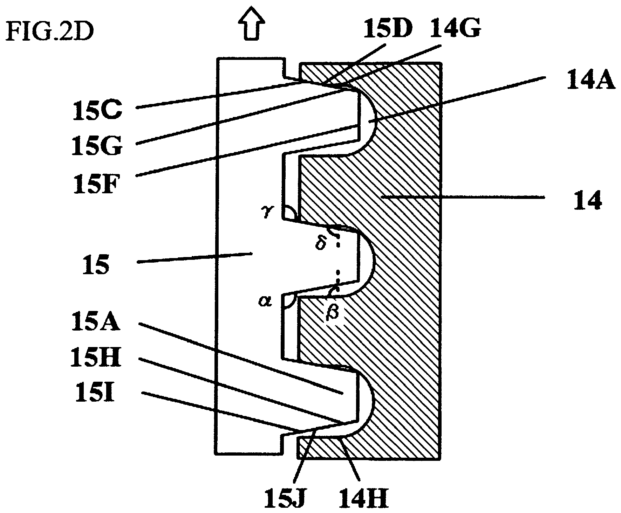

FIGS. 2C and 2D are enlarged schematic views of threadedly-engaged threaded portions of the plunger kit 11 illustrated in FIG. 2B. This plunger kit 11 is an example in which a round screw of a square basic groove profile formed semi-circular in a root part thereof is used as the internal screw 14 of the piston 12 (see FIG. 2B) and a trapezoidal screw of a trapezoidal basic thread profile is used as the external screw 15 of the plunger rod 13 (see FIG. 2B) to be brought into threaded engagement with the round screw. It is to be noted that the term "trapezoidal screw" as used in the present invention shall include screws generally called "triangular screws", in each of which the angle between flanks on opposite sides at a free end of a thread in a basic thread profile is 60° or so. Similarly, the term "round screw" as used in this invention shall include screw in each of which the entire basic thread profile is semi-circular. Needless to say, the terms "trapezoidal, square and round" shall all include their modifications.

In this first embodiment, a root 15E between each two adjacent threads 15A,15A of the external screw 15 extends in the sliding direction of the piston as illustrated in FIG. 2C. A leading flank 15 G of each thread 15A of the external screw 15 has an angle γ (=100°) with respect to the push direction of the piston. On the other hand, a groove flank 14G of the corresponding groove 14A of the internal screw 14, said groove flank 14G being located face-to-face with the leading flank 15 G, has an angle δ (=90°) with respect to the pull direction of the piston. A following flank 15H of each thread 15A of the external screw 15 also has an angle α (=100°) with respect to the pull direction of the piston. On the other hand, a groove flank 14H of the corresponding groove 14A of the internal screw 14, said groove flank 14H being located face-to-face with the following flank 15H, also has an angle β (=90°) with respect to the push direction of the piston. When the external screw 15 and internal screw 14 are brought into threaded engagement with each other, the leading flank 15G and following flank 15H at root-side portions of each thread 15A of the external screw 15 come into contact with the groove flanks 14G of the corresponding groove 14A of the internal screw 14, said groove flanks 14G being located face-to-face with the leading flank 15G and following flank 15H, at and along narrow helical strips, respectively, but the external screw 15 and internal screw 14 remain out of contact at the other areas. Upon sliding of the piston, on the other hand, a load is applied in the sliding direction of the piston on the external screw 15 of the plunger rod so that the internal screw 14 on the side of the piston made of the elastomer undergoes elastic deformation. When the piston is slid in the push direction as indicated by an arrow in FIG. 2D, the leading flank 15G of each thread 15A of the external screw 15, therefore, comes into contact with the groove flank 14G at and along a wide helical strip, the width of which varies corresponding to the load, not only at the root-side portion but also over from the root-side portion toward a crest 15F of the thread 15A. The area and load of the contact increase corresponding to the load applied to the plunger rod.

When the internal screw 14 and external screw 15 are brought into threaded engagement with each other, each leading flank 15G and the groove flank 14G, said groove flank 14G being located face-to-face with the leading flank 15G, have the angles (γ,δ; see FIG. 2C) with respect to the sliding direction, respectively, and these angles are set as a combination of mutually-different angles (γ=100°, δ=90°) , as described above. When the piston is slid in the push direction in the syringe barrel to bring each leading flank 15G into contact with the groove flank 14G, the leading flank 15G, therefore, comes into contact with the groove flank 14G more strongly at a third part 15C than at a fourth part 15D and more weakly at the fourth part 15D than at the third part 15C. In other words, a hard contact part (the third part 15C) and a soft contact part (the fourth part 15D) occur on the leading flank 15G. As a result, the thread engagement strength can be increased between the external screw 15 and the internal screw 14 without any substantial increase in screwing resistance. When the piston is slid in a direction opposite to the arrow indicated in FIG. 2D, namely in the pull direction in the syringe barrel, on the other hand, the following flank 15H of each thread 15A also comes into contact with the groove flank 14H, said groove flank 14H being located face-to-face with the following flank 15H, more strongly at a first part 151 than at a second part 15J and more weakly at the second part 15J than at the first part 151. In other words, a hard contact part (the first part 151) and a soft contact part (the second part 15J) also occur on the following flank 15H. As a result, the thread engagement strength can be increased without any substantial increase in screwing resistance as in the sliding of the piston in the direction of the arrow in FIG. 2D. If the internal screw 14 and external screw 15 are set in profile and dimensions such that a load can be produced between the internal screw 14 and the external screw 15 when they are brought into threaded engagement, in other words, in a state that no external load is applied to the plunger rod, for example, when the width of each thread 15A of the external screw 15 is set greater than the width of the corresponding groove 14A of the internal screw 14, an increased load can be applied on the friction faces of the screws as will be mentioned below, thereby making it possible to further increase the thread engagement strength.

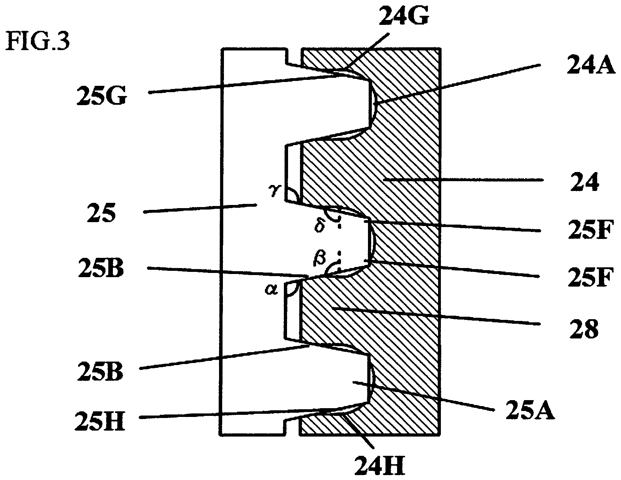

In a second embodiment illustrated by way of example in FIG. 3, angles α, β,γ,δ (α=γ=100°, β=δ=90°) are similar to the angles α,β,γ,δ in FIG. 2C. Similar to the description made above with reference to FIG. 2D, when a piston is slid in a push direction by way of a plunger rod in a syringe barrel, two parts occur on a leading flank 25G of each thread 25A of an external screw 25, one being a part (third part) where the leading flank 25G comes into contact with a groove flank 24G of a corresponding groove 24A of an internal screw 24, said groove flank 24G being located face-to-face with the leading flank 25G, more strongly than at another part (fourth part), and the other being the another part (fourth part) where the leading flank 25G comes into contact with the groove flank 24G more weakly than at the part (third part) . When the piston is slid in a pull direction by way of the plunger rod in the syringe barrel, on the other hand, two parts occur on a following flank 25H of each thread 25A of the external screw 25, one being a part (first part) where the following flank 25H comes into contact with a groove flank 24H, said groove flank 24H being located face-to-face with the following flank 25H, more strongly than at another part (second part), and the other being the another part (second part) where the following flank 25H comes into contact with the groove flank 24H more weakly than at the part (first part). It is, therefore, possible to increase the thread engagement strength between the external screw 25 and the internal screw 24 without any substantial increase in screwing resistance. Further, the internal screw 24 and external screw 25 are set in profile and dimensions such that, when the piston made of an elastomer and the plunger rod made of a harder material than the piston are brought into threaded engagement with each other, each two adjacent threads 25A,25A of the external screw 25 of the plunger rod can come into contact, at root-side portions 25B,25B thereof, with a corresponding inter-groove thread portion 28 of the internal screw 24 of the piston with the inter-groove thread portion 28 being clamped at a free end portion thereof by the root-side portions 25B,25B, to further produce a clamping load between the internal screw 24 and the external screw 25. In the second embodiment described above, the friction force between the internal screw 24 and the external screw 25 is considered to increase still further by the clamping load, and therefore, the thread engagement strength is considered to increase still further.

In the second embodiment illustrated in FIG. 3, not only the root-side portions 25B,25B but also end corner portions 25F,25F of each thread 25A are in strong contact with the groove flanks 24G,24H of the corresponding groove 24A, said groove flanks 24G,24H being located face-to-face with the end corner portions 25F,25F, respectively, by adjusting the height of the thread 25A. The above-described thread engagement strength can also be increased still further by effecting such a modification.

If the friction force between the internal screw 24 and the external screw 25 is increased as described above, another problem, however, arises in that greater screwing resistance is produced upon bringing the piston and plunger rod into threaded engagement with each other. It is, therefore, preferred to set the friction force between the internal screw 24 and the external screw 25 by achieving a balance between mutually-contradictory screwing resistance and thread engagement strength. It is to be noted that the plunger rod does not come out of the piston in a sudden during sliding insofar as the thread engagement strength between the external screw 25 and the internal screw 24 is greater than the sliding resistance of the piston on the inner wall of the syringe barrel.

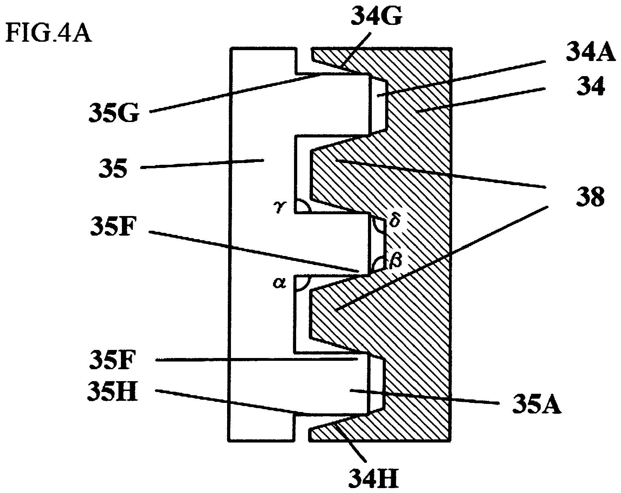

Schematically illustrated in FIGS. 4A through 5C are combined patterns of an internal screw and an external screw in plunger kits according to third to six embodiments of the present invention.

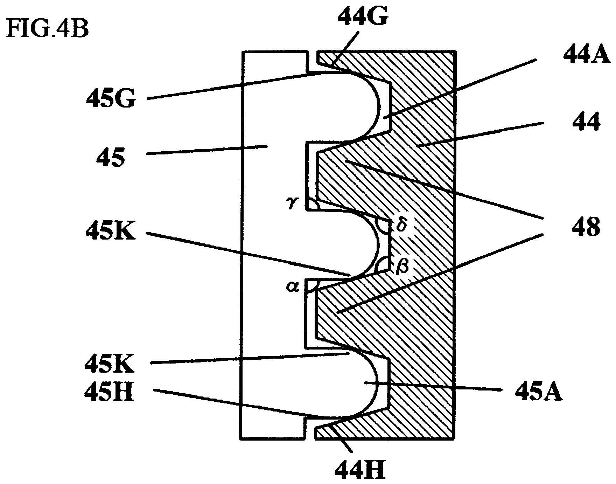

FIG. 4A is an enlarged view illustrating threaded portions of a piston and plunger rod of a plunger kit according to the third embodiment when the piston and plunger rod have been brought into threaded engagement with each other. An internal screw 34 of the piston is a trapezoidal screw that a basic groove profile between each two adjacent, inter-groove thread portions 38,38 is trapezoidal, and an external screw 35 of the plunger rod is a square screw that the basic thread profile of a corresponding thread 35A is rectangular. It is to be noted that the term "square screw" as used in the present invention shall include rectangular screws. FIG. 4B is an enlarged view illustrating threaded portions of a piston and plunger rod of a plunger kit according to the fourth embodiment when the piston and plunger rod have been brought into threaded engagement with each other. An internal screw 44 of the piston is a trapezoidal screw that a basic groove profile between each two adjacent, inter-groove thread portions 48, 48 is trapezoidal, and an external screw 45 of the plunger rod is a round screw that a basic thread profile of a corresponding thread 45A has a rectangular profile a free end part of which is semi-circular.

In each of the combinations of the internal screw and the external screw in FIGS. 4A and 4B, an angle y of a leading flank 35G(45G) of each thread 35A(45A) of the external screw 35(45) with respect to a push direction of the piston and an angle δ of a groove flank 34G(44G) of a corresponding groove 34A(44A) of the internal screw 34(44), said groove flank 34G (44G) being located face-to-face with the leading flank 35G(45G), with respect to a pull direction of the piston when the internal screw 34 (44) and the external screw 35 (45) are brought into threaded engagement with each other are set as a combination of mutually-different angles (γ=90°, 8=105°) . Similar to the description made above with reference to FIG. 2D, when the piston is slid in a push direction, two parts occur on the leading flank 35G (45G), one being a part (third part) where the leading flank 35G (45G) comes into contact with the groove flank 34G (44G) , said groove flank 34G(44G) being located face-to-face with the leading flank 35G (45G), more strongly than at another part (fourth part), and the other being the another part (fourth part) where the leading flank 35G(45G) comes into contact with the groove flank 34G(44G) more weakly than at the part (third part). It is, therefore, possible to increase the thread engagement strength between the external screw 35(45) and the internal screw 34 (44) without any substantial increase in screwing resistance. When the piston is slid in the pull direction by way of the plunger rod in the syringe barrel, on the other hand, two parts occur on a following flank 35H(45H) of each thread 35A(45A) of the external screw 35(45), one being a part (first part) where the following flank 35H (45H) comes into contact with a groove flank 34H (44H), said groove flank 34H(44H) being located face-to-face with the following flank 35H(45H), more strongly than at another part (second part), and the other being the another part (second part) where the following flank 35H (45H) comes into contact with the groove flank 34H (44H) more weakly than at the part (first part) . It is, therefore, possible to increase the thread engagement strength between the external screw 35(45) and the internal screw 34 (44) without any substantial increase in screwing resistance.

Further, each thread 35A(45A) of the external screw 35(45) and its corresponding groove 34A(44A) of the internal screw 34 (44) can be set in profile and dimensions such that each inter-groove thread portion 38(48) of the internal screw 34 (44) can be clamped by end corner portions 35F,35F(intermediate shoulder portions 45K,45K) of adjacent two threads 35A,35A(45A,45A) of the external screw 35(45). A clamping load can, therefore, be produced. The internal screw 34(44) and external screw 35(45) come into contact with each other most strongly at both the end corner portions 35F, 35F (both the intermediate shoulder portions 45K,45K) of each thread 35A(45A). The dimensional difference of the width between both the end corner portions 35F, 35F (both the intermediate shoulder portions 45K,45K) from the width of the groove 34A(44A) at both the end corner portions 35F, 35F (both the intermediate shoulder portions 45K, 45K) can be determined based on a balance between the above-described screwing resistance and thread engagement strength. According to a study by the present inventor, the screwing resistance and thread engagement strength can be well-balanced between the external screw 35(45) and the internal screw 34(44) when in the case of a piston, for example, as small as 13 mm in diameter and 10 mm in height or so, the above-described dimensional difference is designed such that into both the groove flanks 34G,34H(44G,44H) of each groove 34A(44A) of the internal screw 34(44), the end corner portions 35F,35F (both the intermediate shoulder portions 45K,45K) of the corresponding thread 35A(45A) of the external screw 35(45) can be pressed as much as preferably from 0.05 to 0.50 mm or so, more preferably from 0.10 to 0.30 mm or so. It is to be noted that these numeral values were obtained when chlorinated butyl rubber and polypropylene were adopted as the material of the piston and the material of the plunger rod, respectively. These numerical values can be equally applied when the combined profiles of the internal screw and external screw are reversed.

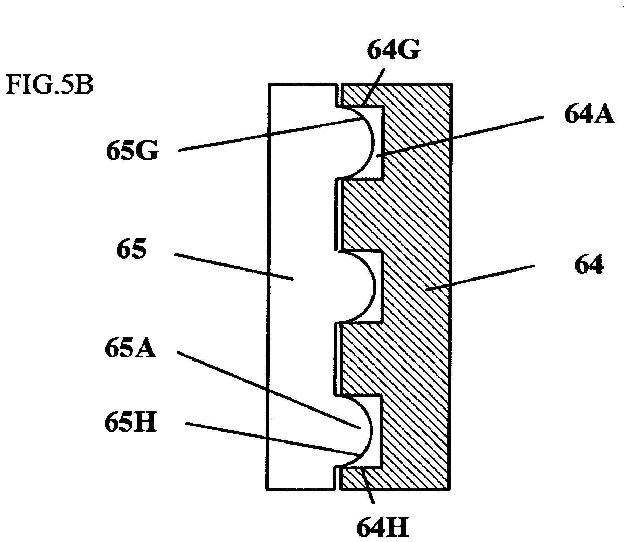

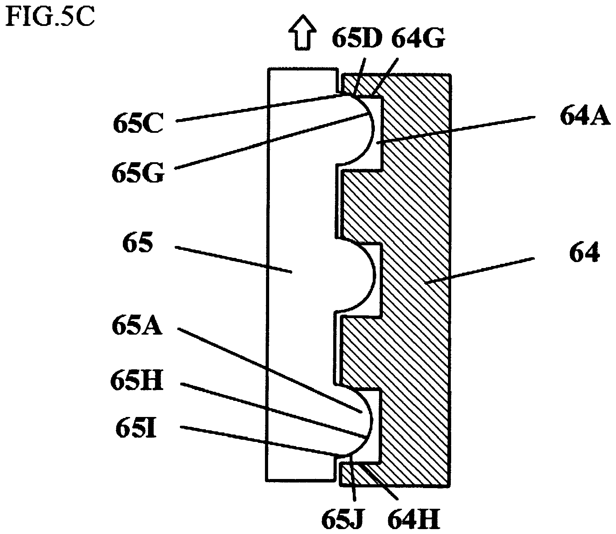

In the combination of the internal screw and the external screw in FIG. 4B, the angle γ of the leading flank 45G of each thread 45A of the external screw 45 with respect to the push direction of the piston and the angle δ of the groove flank 44G of the corresponding groove 44A of the internal screw 44, said groove flank 44G being located face-to-face with the leading flank 45G, with respect to the pull direction of the piston when the internal screw 44 and the external screw 45 are brought into threaded engagement with each other are set as a combination of mutually-different angles (γ=90°,δ=105°) as described above. Further, when the internal screw 44 and the external screw 45 are brought into threaded engagement with each other, a free end part of the leading flank 45G of each thread 45A of the external screw 45 is curved and the groove flank 44G of the corresponding groove 44A of the internal thread 44, said groove flank 44G being located face-to-face with the leading flank 45G, is flat, so that the leading flank 45G and the groove flank 44G are also set as a combination of a flat surface and a curved surface. The term "flat surface" as used in the present invention means each surface shown by a straight line among the leading flank and following flank (which may hereinafter be called "thread flanks") of each thread and the groove flanks of the groove corresponding to the thread, said groove flanks being located face-to-face with the thread flanks, respectively. The term "curved surface", on the other hand, means each surface shown by a curved line among the thread flanks and groove flanks. As such curved surfaces, the above-mentioned thread flanks or groove flanks in each of FIGS. 2A to 2D, FIG. 3 and FIG. 4B have curved surfaces only at the parts thereof, but in FIGS. 5B and 5C to be described subsequently herein, the thread flanks or groove flanks may have curved surfaces at the entire parts thereof.

When each thread flank and a groove flank, which is located face-to-face with the thread flank, are set as a combination of a flat surface and a curved surface like the combination of the internal screw and external screw illustrated in each of FIGS. 2A to 2D, FIG. 3 and FIG. 4B upon threaded engagement of an internal screw and external screw, a hard contact part and a soft contact part occur on the thread flank when a load is applied to the external screw by sliding a piston in a syringe barrel. Similar to the above-mentioned combination of a thread flank and a groove flank, said glove flank being located face-to-face with the thread flank, which have mutually-different angles with respect to the sliding direction of the piston, it is, therefore, possible to increase the thread engagement strength between the external screw and the internal screw without any substantial increase in screwing resistance.

In the combination of a flat surface and a curved surface, whichever of the flat surface and the curved surface can be either on the side of an external screw or on the side of an internal screw. Preferably, however, the curved surface may exist on the side of the external screw of the plunger rod made of a hard material as illustrated in FIG. 4B and FIGS. 5B and 5C to be described subsequently herein because, even when a load is applied, the curved surface is believed to be resistant to deformation and the effect of the combination of the flat surface and curved surface is believed to remain highly.

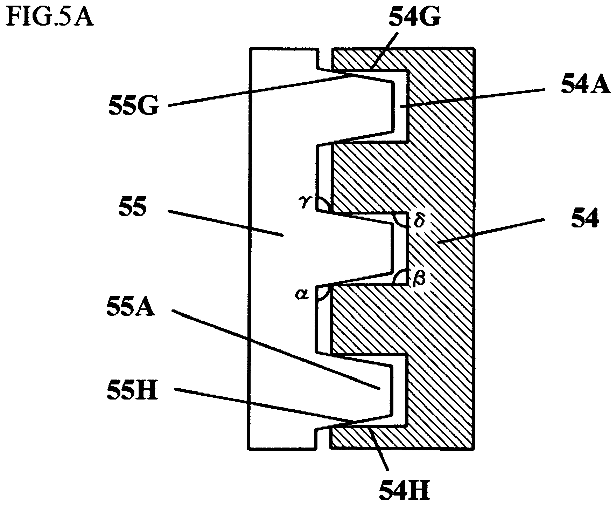

In each of FIG. 5A and FIG. 5B, an internal screw 54 (64) is a square screw, the basic groove profile of which is square or rectangular, like the above-described external screws 35,45 in FIG. 4A and FIG. 4B. On the other hand, an external screw 55 in FIG. 5A is a trapezoidal screw the basic thread profile of which is trapezoidal, and an external screw 65 in FIG. 5B is a round screw the basic thread profile of which is semi-circular in its entirety.

In FIG. 5A, an angle γ of a leading flank 55G of each thread 55A of the external screw 55 with respect to a push direction of a piston and an angle δ of a groove flank 54G of the corresponding groove 54A of the internal screw 54, said groove flank 54G being located face-to-face with the leading flank 55G, with respect to a pull direction of the piston when the internal screw 54 and the external screw 55 are brought into threaded engagement with each other are set as a combination of mutually-different angles (γ=100°, δ=90°) . Similar to the description made above with reference to FIG. 2D, when the piston is slid in the push direction, two parts occur on the leading flank 55G, one being a part (third part) where the leading flank 55G comes into contact with the groove flank 54G, said groove flank 54G being located face-to-face with the leading flank 55G, more strongly than at another part (fourth part), and the other being the another part (fourth part) where the leading flank 55G comes into contact with the groove flank 54G more weakly than at the part (third part). It is, therefore, possible to increase the thread engagement strength between the external screw 55 and the internal screw 54 without any substantial increase in screwing resistance. When the piston is slid in the pull direction by way of the plunger rod in the syringe barrel, on the other hand, two parts occur on a following flank 55H of each thread 55A of the external screw 55, one being a part (first part) where the following flank 55H comes into contact with a groove flank 54H, said groove flank 54H being located face-to-face with the following flank 55H, more strongly than at another part (second part), and the other being the another part (second part) where the following flank 55H comes into contact with the groove flank 54H more weakly than at the part (first part). It is, therefore, possible to increase the thread engagement strength between the external screw 55 and the internal screw 54 without any substantial increase in screwing resistance.

In FIG. 5B, there is the combination of a square screw as the internal screw 64 and a round screw as the external screw 65 as described above. When the internal screw 64 and the external screw 65 are brought into threaded engagement with each other, a leading flank 65G of each thread 65A of the external screw 65 has a curved surface and a groove flank 64G of a corresponding groove 64A of the internal thread 64, said groove flank 64G being located face-to-face with the leading flank 65G, has a flat surface. When a piston is slid in a syringe barrel in the direction of an arrow as indicated in FIG. 5C, in other words, in a push direction, two parts occur on the curved surface of the leading flank 65G of each thread 65A as in the description made above with reference to FIG. 2D, one being a part (third part) 65C where the curved surface comes into contact with the flat surface of the groove flank 64G of the corresponding groove 64A, said flat surface being located face-to-face with the leading flank 65G, more strongly than at another part (fourth part) 65D, and the other being the another part (fourth part) 65D where the curved surface comes into contact with the flat surface of the groove flank 64G more weakly than at the part (third part) 65C. It is, therefore, possible to increase the thread engagement strength between the external screw 65 and the internal screw 64 without any substantial increase in screwing resistance as in the combination of the mutually-different angles (γ,δ) in FIG. 5A. When the piston is slid in a pull direction by way of the plunger rod in the syringe barrel, on the other hand, two parts occur on a following flank 65H of each thread 65A of the external screw 65 as in the description made above with reference to FIG. 2D, one being a part (first part) 65I where the following flank 65H comes into contact with a groove flank 64H, said groove flank 64H being located face-to-face with the following flank 65H, more strongly than at another part (second part) 65J, and the other being the another part (second part) 65J where the following flank 65H comes into contact with the groove flank 64H more weakly than at the part (first part) 65I. When the external screw is a round screw a free end part of the basic thread profile of which is semi-circular as illustrated in FIG. 4B although this construction is not illustrated in any figure, similar advantageous effects can be also brought about obviously. Although not illustrated in any figure either, similar advantageous effects can also be brought about with a combination of a round screw as an external screw and a square screw as an external screw.

It is to be noted that the combination of an internal screw and an external screw in the present invention is not necessarily limited to the above-described combinations. It is possible to combine, for example, a modified-profile screw the profile of which is totally different from a normal trapezoidal screw, square screw or the like. Even if an external screw and an internal screw are both trapezoidal screws, opposite groove flanks of each groove of the internal screw and opposite thread flanks of a corresponding thread of the external screw, said thread flanks being located face-to-face with the groove flanks upon threaded engagement, can be set as a combination of mutually-different angles with respect to a sliding direction of a piston as in the above-described first to fifth embodiments provided that the external screw and internal screw are different in the inclination of the sloping sides of the trapezoid. On each thread flank, a hard contact part and a soft contact part can, therefore, occur to the groove flank located face-to-face with the thread flank.

In each of the above-described embodiments, the basic groove profile and basic thread profile are each bilaterally symmetrical. In other words, the groove flanks on opposite sides of the basic groove profile and the thread flanks, specifically the leading flank and following flank on opposite sides of the basic thread profile are bilaterally symmetrical. In the present invention, however, these basic groove profile and basic thread profile may each be bilaterally asymmetrical. In the case of a trapezoidal screw thread form, its trapezoid may hence be either isosceles or anisosceles.

Portions where a clamping load is to be produced may preferably be root-side portions of each thread of an external screw, in other words, free end portions of each inter-groove thread portion of an internal screw. Because, even if deformation occurs at a free end portion of the piston, the free end portion is distant to the outer circumference of the piston and the effects of the deformation hardly appear on the outer circumference. If the effects of deformation by clamping appear on the outer circumference of the piston, the sliding resistance may increase, or in a severe case, the sealing performance of the piston may be impaired to cause a leakage problem.

Piston materials usable in the present invention can include those which are elastomers and have conventionally been employed as materials for pistons of syringes for use with medicines. Illustrative synthetic rubbers include butyl rubber (IIR), chlorinated butyl rubber (CIIR), brominated butyl rubber (BIIR), partially-crosslinked IIR, polybutadiene rubber (BR), polyisoprenes rubber (IR), ethylene-propylene-diene ternary copolymer rubber (EPDM), styrene-butadiene copolymer rubber (SBR), acrylic rubber (ACM), acrylonitrile butadiene rubber (NBR), and the like. Illustrative thermoplastic elastomers include polyisobutylene-based thermoplastic elastomers (SIBS), styrene-based elastomers such as styrene-butadiene-styrene(SBS)-based copolymers, styrene-ethylene-butylene-styrene(SEBS)-based copolymers and styrene-isoprene-styrene(SIS)-based copolymers, and olefin-based elastomers such as ethylene-propylene-nonconjugated diene monomer(EPDM)-based copolymers and ethylene-propylene(EPM)-based copolymers. Among these, butyl rubber, chlorinated butyl rubber and polyisobutylene-based elastomers are preferred for gas barrier performance, dissolution characteristics and the like. Preferably, pistons may be laminated at their sliding surfaces with syringe barrels and their top walls, where they come into contact with medical solutions, with fluorinated resin films such as PTFE films.

Plunger rod shapes usable in the present invention can include, in addition to the cylindrical shape illustrated in FIGS. 2A and 2B, those having cross-shaped ribs in cross-section, those having H-shaped ribs in cross-section, and the like, all of which have conventionally been used in plunger rods. From the standpoint of reducing shaking of a plunger rod in a direction lateral to its sliding direction, a cylindrical plunger rod materials can include those which are harder than associated pistons and have conventionally been employed as materials for plunger rods of syringes for use with medicines. As synthetic resins, illustrative are general-purpose plastics such as polyethylene and polypropylene.

The dimensions of each piston for use in the present invention is 13 mm or smaller in diameter and 10 mm or smaller in height, in other words, the dimensions of the internal screw of each piston may preferably be about 10 mm or smaller in diameter and about 7 mm or smaller in length. The advantageous effects of the present invention can be still observed on plunger kits making use of pistons having greater dimensions that the above-mentioned dimensions, but in many instances, they often have sufficient thread engagement strength without needing practicing the present invention. The dimensions of the smallest piston which is producible in mass quantities at present are about 3 mm or so in diameter and about 4 mm or so in height, and as the dimensions of its internal screw, about 2 mm or so in diameter and about 2 mm or so in length. The present invention can bring about more pronounced advantageous effects when applied to such small plunger kits useful in syringes of ever reduced capacity.

Upon practicing the present invention, it is also effective to change one or more of the dimensions of grooves and threads in addition to combining the above-mentioned internal screw and external screw of different profiles. For example, the width and height of threads can be set smaller than those of grooves, and the resulting reduction in the thread engagement strength between them can be compensated for by the advantageous effects of the present invention. The above-mentioned changes can lessen the potential deterioration of sliding performance due to an expansion of the piston and can also reduce the screwing resistance while maintaining the thread engagement strength as it is.

However, a change in profile such as making the height of threads greater than the depth of grooves results in expanding the internal screw of the piston by the external screw of the plunger rod. Such a change in profile involves a potential problem of an increase in the sliding resistance of the piston, and therefore, is not preferred obviously.

The screw construction according to the present invention is particularly effective when the grooves and threads are each of the same profile from their starts to the ends, but only portions of the grooves and threads may be formed in such profiles as in the present invention, respectively. To assure the availability of the effects of the present invention, however, 60% or more, preferably 80% or more of the entire internal screw and external screw may preferably be formed of the combination of grooves and threads according to the present invention.

Further, thread(s) may preferably be arranged to circle at least once, desirably two to four times around a cylinder. This equally applies to groove(s).

The present invention will hereinafter be described in further detail based on examples and comparative examples.

### Example 1

With chlorinated butyl rubber ("HT1066", trade name, product of Exxon Mobile Corporation), a piston of the shape illustrated in FIGS. 2A to 2D was produced. The piston was about 13 mm in diameter and about 10 mm in height, and was provided with an internal screw in the form of a round screw having a square basic groove profile which was formed semi-circular in a root part thereof. The piston was laminated at its sliding surface with a syringe barrel and its top wall, where it would come into contact with a medical solution, with a PTFE film ("TEFLON 7A", trade name, product of E.I. du Pont de Nemours and Company) . As dimensions of the internal screw, its cylinder space was set at 5.0 mm in diameter and 7.0 mm in length, and in the basic groove profile, its grooves were set at 2.0 mm in depth at deepest parts thereof from the cylinder space and 1.0 mm in width at shallowest parts thereof from the cylinder space.

To be combined with the piston, a plunger rod of the shape illustrated in FIGS. 2A to 2D was also produced with polypropylene ("HIZEX", trade mark, product of Mitsui Chemicals, Inc.). The plunger rod was about 10 mm in diameter and about 50 mm in length, and was provided with an external screw the basic thread profile of which was trapezoidal. As dimensions of the external screw, its cylinder was set at 4.9 mm in diameter and 7.0 mm in length, and in the basic thread profile, its threads were set at 2.0 mm in height at tallest parts (crests) thereof from the surface of the cylinder, 0.8 mm in width at the crest thereof, and 1.2 mm in width at roots thereof. The threads were arranged to circle three times around the cylinder at a uniform pitch over the entire length thereof. The grooves were arranged similarly.

### Example 2

A piston and plunger rod were produced in a similar manner as in Example 1 except that as illustrated in FIG. 4A, the basic groove profile of the internal screw and the basic thread profile of the external screw were changed to a trapezoidal profile and a rectangular profile, respectively, and the dimensions of the internal screw and external screw were set as will be described below. As the dimensions of the internal screw, its cylinder space was set at 7.0 mm in diameter, and its grooves were set at 2.0 mm in depth at deepest parts thereof from the cylinder space, 0.8 mm in width at the deepest parts thereof from the cylinder space, and 1.2 mm in width at shallowest parts thereof from the cylinder space. As dimensions of the external screw, on the other hand, its cylinder was set at 6.7 mm in length, and its threads were set at 2.0 mm in height and 1.0 mm in width.

### Example 3

A plunger kit was produced in a similar manner as in Example 1 except that as illustrated in FIG. 4B, a piston similar to that of Example 2 was used, the basic thread profile of a plunger rod to be combined with the piston was changed to a rectangular profile a free end part of which was semi-circular, and its dimensions were set as will be described below. As the dimensions of the external screw of the plunger rod, its cylinder was set at 6.7 mm in length, and its threads were set at 2.0 mm in height and at 1.0 mm in width at the parts thereof other than the free end parts thereof. Further, the free end parts of the threads were rounded off at the corners thereof to form them into semi-circles of 1.0 mm in diameter.

### Example 4

A plunger kit was produced in a similar manner as in Example 1 except that a plunger rod similar to that of Example 3 was used, the basic groove profile of a piston to be combined with the plunger rod was changed to a rectangular profile, and its dimensions were set as will be described below. As the dimensions of the internal screw of the piston, its cylinder space was set at 7.0 mm in diameter and 7.0 mm in length, and its grooves were set at 2.0 mm in depth and 1.0 mm in width.

### Examples 5 to 7

Plunger rods were produced in exactly the same manner as in Examples 1 to 3, respectively, except that the widths of threads of the external screws of the plunger rods were increased by 0.2 mm in their entirety. The plunger rods were combined with pistons similar to those employed in Examples 1 to 3, respectively, to provide plunger kits of Examples 5 to 7.

### Comparative Example 1

A piston and plunger rod, the basic groove profile and basic thread profile of which were both trapezoidal, were produced in a similar manner as in Example 1, and were provided as a plunger kit of Comparative Example 1. The dimensions of its internal screw and external screw were the same except that the cylinder space was 7.0 mm in diameter and the cylinder was 6.7 mm in diameter. The threads and grooves were set at 2.0 mm in height and depth, respectively, the grooves and threads were set at 0.8 mm in width at deepest parts thereof and tallest parts thereof, respectively, and the grooves and threads were set at 1.2 mm in width at shallowest parts thereof and roots thereof, respectively.

### Comparative Example 2

In a similar manner as in Comparative Example 1, a piston and plunger rod, the basic groove profile and basic thread profiles of which were both square, were produced and were provided as a plunger kit of Comparative Example 2. The dimensions of its internal screw and external screw were the same except that the cylinder space was 7.0 mm in diameter and the cylinder was 6.7 mm in diameter. The threads and grooves were set at 2.0 mm in height and depth, respectively, and the grooves and threads were both set at 1.0 mm in width.

### Comparative Examples 3 and 4

Plunger rods were produced in exactly the same manner as in Comparative Examples 1 and 2, respectively, except that the widths of threads of the external screws of the plunger rods were increased by 0.2 mm in their entirety. The plunger rods were combined with pistons similar to those employed in Comparative Examples 1 and 2, respectively, to provide plunger kits of Comparative Examples 3 and 4.

### Evaluation

### Screwing Resistance Test

Each piston produced as described above was inserted in a syringe barrel of about 12 mm in diameter. With the syringe barrel being fixed, the corresponding plunger rod was brought into threaded engagement with the piston in the syringe by hand. Resistance at that time was measured and presented in Table 1 and Table 2. Each plunger kit was ranked "A" when the resistance was small, "B" when the resistance was a little large although it posed no problem in use, or "C" when the resistance was so large that it posed a problem in use. The number of test samples was set at 10, and the ranking was performed based on their average. The ranking results are also presented in Table 1 and Table 2.

### Sliding Resistance Test

The pistons produced as described above were inserted in syringe barrels for 5 mL capacity (inner diameter: about 12 mm; "CZ RESIN", trade name, product of Daikyo Seiko, Ltd.), respectively, and portions (specified volume: 5 mL) of water were filled in the respective syringe barrels. The syringe barrels, together with the pistons inserted and water filled therein, were allowed to stand for 12 hours. Subsequently, the piston in each syringe was brought into threaded engagement with the corresponding plunger rod, and an injection needle of 21G was fitted in a nozzle of the syringe barrel. The plunger rod was pushed in at a speed of 100 mm/min, and the maximum value (N) of sliding resistance during that time was measured. The maximum values (N) of sliding resistance obtained as described above are also presented in Table 1 and Table 2. The plunger rod was then pulled out at the same speed and also at a speed of 200 mm/min to the position before the initiation of the test to confirm whether or not the threaded engagement of the piston with the plunger rod would be maintained. The evaluation was performed by counting the number of samples in which the threaded engagement was maintained. For the measurement, a precision universal tester ("AUTOGRAPH AG-5KNIS MS", trade name, manufactured by Shimadzu Corporation; load cell: 100 N) was used as a tensile tester. The number of test samples was set at 10 for each pull-out speed, and the ranking was performed based on their average. The ranking results are also presented in Table 1 and Table 2.

**Table 1 Evaluation Results of Examples**

| | Ex. 1 | Ex. 2 | Ex. 3 | Ex. 4 | Ex. 5 | Ex. 6 | Ex. 7 |
|---|---|---|---|---|---|---|---|
| Sliding resistance (N) | 7.8 | 7.8 | 7.8 | 7.7 | 7.8 | 9.8 | 8.6 |
| | 8.0 | 8.1 | 7.5 | 7.7 | 8.4 | 9.5 | 10.1 |
| | 8.1 | 8.2 | 8.2 | 7.4 | 8.2 | 10.2 | 9.4 |
| | 7.8 | 7.9 | 7.6 | 8.0 | 7.7 | 9.8 | 9.2 |
| | 7.4 | 8.1 | 8.4 | 8.1 | 8.8 | 11.0 | 10.3 |
| | 7.7 | 7.6 | 8.0 | 8.3 | 8.1 | 10.7 | 8.6 |
| | 8.3 | 7.7 | 7.4 | 8.0 | 8.0 | 10.6 | 8.4 |
| | 7.9 | 7.5 | 7.9 | 7.9 | 8.1 | 10.8 | 9.1 |
| | 7.5 | 8.1 | 7.7 | 8.0 | 7.8 | 9.9 | 9.4 |
| | 7.9 | 7.8 | 7.6 | 7.8 | 8.0 | 10.4 | 9.8 |
| Average | 7.8 | 7.9 | 7.8 | 7.9 | 8.1 | 10.3 | 9.3 |
| Screwing resistance | A | A | A | B | B | B | B |
| Samples with threaded engagement maintained at: | | | | | | | |
| 100* | 10/10 | 10/10 | 10/10 | 10/10 | 10/10 | 10/10 | 10/10 |
| 200* | 9/10 | 8/10 | 9/10 | 8/10 | 10/10 | 10/10 | 10/10 |

| | | | | | | | |
|---|---|---|---|---|---|---|---|
| * Unit of testing speed for the maintenance of threaded engagement: mm/min | | | | | | | |

**Table 2 Evaluation Results of Comparative Examples**

| | Comp. Ex. 1 | Comp. Ex. 2 | Comp. Ex. 3 | Comp. Ex. 4 |
|---|---|---|---|---|
| Sliding resistance (N) | 7.8 | 7.8 | 11.0 | 15.2 |
| | 7.6 | 8.0 | 11.8 | 14.8 |
| | 7.6 | 8.1 | 10.8 | 15.5 |
| | 8.1 | 7.7 | 11.5 | 15.0 |
| | 7.6 | 7.5 | 11.2 | 15.3 |
| | 8.2 | 7.6 | 11.5 | 14.8 |
| | 8.3 | 8.1 | 11.5 | 15.6 |
| | 8.0 | 8.3 | 10.9 | 15.2 |
| | 7.8 | 7.6 | 11.2 | 15.0 |
| | 7.7 | 7.5 | 11.4 | 15.3 |
| Average | 7.9 | 7.8 | 11.3 | 15.2 |
| Screwing resistance | B | B | C | C |
| Samples with threaded engagement maintained at: | | | | |
| 100* | 8/10 | 9/10 | 9/10 | 10/10 |
| 200* | 5/10 | 6/10 | 7/10 | 7/10 |

| | | | | |
|---|---|---|---|---|
| * Unit of testing speed for the maintenance of threaded engagement: mm/min | | | | |

## Claims

1. A plunger kit as a component for a medicine syringe provided with a syringe barrel and useful for injecting a medicine, said plunger kit comprising a piston (12) having a diameter of 13 mm or smaller and a height of 10 mm or smaller made of an elastomer and having an internal screw (14;24;34;44;54;64) and a plunger rod (13) made of a material harder than the piston and having an external screw (15;25;35;45;55;65);
wherein the internal screw and external screw have a mutually non-complementary, basic groove profile and basic thread profile, respectively; and
a following flank (15H;25H;35H;45H;55H;65H) of each thread (15A;25A;35A;45A;55A;65A) of the external screw and a groove flank (14H;24H;34H;44H;54H;64H) of a corresponding groove (14A;24A;34A;44A;54A;64A) of the internal screw are formed such that, when the piston and plunger rod are brought into threaded engagement with each other and slidably fit in the syringe barrel and the piston is then slid in a pull direction by way of the plunger rod, the following flank can come into contact with the groove flank, which is located face-to-face with the following flank, more strongly at a first part (15I;65I) of the following flank than at a second part (15J;65J) of the following flank and more weakly at the second part than at the first part to increase thread engagement strength between the external screw and the internal screw without any substantial expansion of the piston by the external screw;
and wherein the angle (α) of the following flank with respect to the pull direction of the piston and the angle (β) of the groove flank with respect to the push direction of the piston are each from 90 to 150°, and the angles are different from each other.

2. The plunger kit according to claim 1, wherein one (15A;25A;34A;44A;55A) of the basic groove profile of the internal screw and the basic thread profile of the external screw has a trapezoidal screw thread form, and the other (14A;24A;45A;35A;54A) has one of a round thread form, a square thread form and another trapezoidal screw form having sloping sides inclinations of which are different from those of sloping sides of the first-mentioned trapezoidal screw thread form.

3. The plunger kit according to claim 1, wherein one (15H;25H;44H;64H) of the following flank and the groove flank, which is located face-to-face with the following flank, has a flat surface, and the other (14H;24H;45H;65H) has a curved surface.

4. The plunger kit according to claim 3, wherein the curved surface is formed on at least a part of the following flank (45H;65H).

5. The plunger kit according to claim 3 or 4 , wherein one (14A;24A;45A;65A) of the basic groove profile of the internal screw and the basic thread profile of the external screw has a round thread form, and the other (15A;25A;44A;64A) has a trapezoidal screw thread form or a square thread form.

6. The plunger kit according to any one of claims 1-5, wherein a leading flank (15G;25G;35G;45G;55G;65G) of each thread (15A;25A;35A;45A;55A;65A) of the external screw (15;25;35;45;55;65) and an opposite groove flank (14G;24G;34G;44G;54G;64G) of the corresponding groove (14A;24A;34A;44A;54A;64A) of the internal screw (14;24;34;44;54;64) are formed such that, when the piston (12) is slid in a push direction by way of the plunger rod (13) in the syringe barrel, the leading flank can come into contact with the opposite groove flank, which is located face-to-face with the leading flank, more strongly at a third part (15C;65C) of the leading flank than at a fourth part (15D;65D) of the leading flank and more weakly at the fourth part than at the third part to increase thread engagement strength between the external screw and the internal screw without any substantial expansion of the piston by the external screw.

7. The plunger kit according to any one of claims 1-6, wherein the external screw (25) and internal screw (24) are set in profile and dimensions such that each two adjacent threads (25A,25A) of the external screw can come into contact, at root-side portions (25B,25B) thereof, with an inter-groove thread portion (28) of the internal screw, said inter-groove thread portion being located between the two threads, with the inter-groove thread portion being clamped at a free end portion thereof by the root-side portions, to produce a clamping load between the internal screw and the external screw.

8. The plunger kit according to any one of claims 1-6, wherein each thread (25A;35A) of the external screw (25;35) and its corresponding groove (24A;34A) of the internal screw (24;34) are set in profile and dimensions such that the thread can come into contact, at end corner portions (25F,25F;35F,35F) thereof located between a crest thereof and a leading flank (25G;35G) and following flank (25H;35H) thereof, respectively, with groove flanks (24G,24H;34G,34H) of the groove, said groove flanks being located face-to-face with the leading flank and following flank, respectively, to produce a clamping load between the internal screw and the external screw.

9. The plunger kit according to any one of claims 1-6, wherein each thread (45A) of the external screw (45) is provided, on intermediate parts of a leading flank (45G) and following flank (45H) thereof, with shoulder portions (45K,45K), respectively, and the thread (45A) of the external screw and the corresponding groove (44A) of the internal screw are set in profile and dimensions such that the shoulder portions can come into contact with the groove flanks (44G,44H) of the groove, respectively, to produce a clamping load between the internal screw and the external screw.

10. The plunger kit according to any one of claims 1-9, wherein the piston (12) has the internal screw (14) threadedly engageable with the plunger rod (13), the internal screw defines therein a cylinder space (14B) and grooves (14A) helically extending from the cylinder space toward an outer circumference of the piston, the plunger rod has the external screw (15) threadedly engageable with the piston, and the external screw has a cylinder (15B) and threads (15A) helically extending in a radial and outward direction from the cylinder.

11. A medicine syringe **characterized by** comprising a syringe barrel and the plunger kit according to any one of claims 1-10 slidably fitted in a threadedly-engaged form in the syringe barrel.

## Patentansprüche

1. Kolbensatz als Bestandteil für eine Arzneimittelspritze, die mit einem Spritzenzylinder bereitgestellt wird und zum Injizieren eines Arzneimittels geeignet ist, wobei der Kolbensatz einen Kolben (12) umfasst, der einen Durchmesser von 13 mm oder weniger und eine Höhe von 10 mm oder weniger aufweist, der aus einem Elastomer besteht und eine innere Schraube (14; 24; 34; 44; 54; 64) aufweist und eine Kolbenstange (13) aus einem Material, das härter als der Kolben ist und eine äußere Schraube (15; 25; 35; 45; 55; 65) aufweist;
wobei die innere Schraube und die äußere Schraube ein gegenseitiges nichtkomplementäres grundlegendes Rillenprofil beziehungsweise ein grundlegendes Gewindeprofil aufweisen; und
eine folgende Flanke (15H; 25H; 35H; 45H; 55H; 65H) jedes Gewindes (15A; 25A; 35A; 45A; 55A; 65A) der äußeren Schraube und eine Rillenflanke (14H; 24H; 34H; 44H; 54H; 64H) einer entsprechenden Rille (14A; 24A; 34A; 44A; 54A; 64A) der inneren Schraube derart ausgebildet sind, dass, wenn der Kolben und die Kolbenstange in Gewindeeingriff miteinander gebracht werden und verschiebbar in den Spritzenzylinder eingepasst sind und der Kolben dann mittels der Kolbenstange in Zugrichtung geschoben wird, die folgende Flanke mit der Rillenflanke, die sich gegenüber der folgenden Flanke befindet, stärker mit dem ersten Teil (15I, 65I) der folgenden Flanke als mit dem zweiten Teil (15J; 65J) der folgenden Flanke, und schwächer am zweiten Teil als am ersten Teil, in Kontakt kommen kann, um die Stärke des Gewindeeingriffs zwischen der äußeren Schraube und der inneren Schraube ohne wesentliche Ausdehnung des Kolbens durch die äußere Schraube zu erhöhen; und wobei der Winkel (α) der folgenden Flanke in Bezug auf die Zugrichtung des Kolbens und der Winkel (β) der Rillenflanke in Bezug auf die Druckrichtung des Kolbens jeweils von 90 bis 150° betragen, und sich die Winkel voneinander unterscheiden.

2. Kolbensatz nach Anspruch 1, wobei eines (15A; 25A; 34A; 44A; 55A), das grundlegende Rillenprofil der inneren Schraube und das grundlegende Gewindeprofil der äußeren Schraube ein Trapezgewinde aufweist, und das andere (14A; 24A; 45A; 35A; 54A) ein Rundgewinde, ein Flachgewinde und ein anderes Trapezgewinde mit geneigten Seiten aufweist, deren Neigungen sich von denen der geneigten Seiten des erstgenannten Trapezgewindes unterscheiden.

3. Kolbensatz nach Anspruch 1, wobei eine (15H; 25H; 44H; 64H), die folgenden Flanke und die Rillenflanke, die sich gegenüber der folgenden Flanke befindet, eine flache Oberfläche aufweist und die andere (14H; 24H; 45H; 65H) eine gekrümmte Oberfläche aufweist.

4. Kolbensatz nach Anspruch 3, wobei die gekrümmte Oberfläche auf wenigstens einem Teil der folgenden Flanke (45H; 65H) ausgebildet ist.

5. Kolbensatz nach Anspruch 3 oder 4, wobei eines (14A; 24A; 45A; 65A), das grundlegende Rillenprofil der inneren Schraube und das grundlegende Gewindeprofil der äußeren Schraube, ein Rundgewinde aufweist und das andere (15A; 25A; 44A; 64A) ein Trapezgewinde oder ein Flachgewinde aufweist.

6. Kolbensatz nach einem der Ansprüche 1 bis 5, wobei die Vorderflanke (15G; 25G; 35G; 45G; 55G; 65G) jedes Gewindes (15A; 25A; 35A; 45A; 55A; 65A) der äußeren Schraube (15; 25; 35; 45; 55; 65) und die gegenüberliegende Rillenflanke (14G; 24G; 34G; 44G; 54G; 64G) der entsprechenden Rille (14A; 24A; 34A; 44A; 54A; 64A) der inneren Schraube (14; 24; 34; 44; 54; 64) derart ausgebildet sind, dass, wenn der Kolben (12) in Druckrichtung mittels der Kolbenstange (13) in dem Spritzenzylinder geschoben wird, die Vorderflanke mit der gegenüberliegenden Rillenflanke, die sich gegenüber der Vorderflanke befindet, stärker an einem dritten Teil (15C; 65C) der Vorderflanke als an einem vierten Teil (15D; 65D) der Vorderflanke, und schwächer am vierten Teil als am dritten Teil, in Kontakt kommen kann, um die Stärke des Gewindeeingriffs zwischen der äußeren Schraube und der inneren Schraube ohne wesentliche Ausdehnung des Kolbens durch die äußere Schraube zu erhöhen.

7. Kolbensatz nach einem der Ansprüche 1 bis 6, wobei die äußeren Schraube (25) und die inneren Schraube (24) in Profil und Abmessungen derart eingestellt sind, dass jeweils zwei benachbarte Gewinde (25A, 25A) der äußeren Schraube an den Abschnitten der Grundseite (25B, 25B) derselben mit dem Gewindeabschnitt zwischen den Rillen (28) der inneren Schraube in Kontakt kommen können, wobei sich der Gewindeabschnitt zwischen den Rillen zwischen den beiden Gewinden befindet, wobei der Gewindeabschnitt zwischen den Rillen an seinem freien Endabschnitt durch die Abschnitte der Grundseite festgeklemmt wird, um eine Klemmkraft zwischen der inneren Schraube und der äußeren Schraube zu erzeugen.

8. Kolbensatz nach einem der Ansprüche 1 bis 6, wobei jedes Gewinde (25A; 35A) der äußeren Schraube (25; 35) und dessen entsprechende Rille (24A; 34A) der inneren Schraube (24; 34) in Profil und Abmessungen derart eingestellt sind, dass das Gewinde an den Enden der Eckenabschnitte (25F, 25F; 35F, 35F) desselben, die sich zwischen einem Scheitel derselben und einer Vorderflanke (25G; 35G) beziehungsweise der folgenden Flanke (25H; 35H) befinden, mit den Rillenflanken (24G, 24H; 34G, 34H) der Rille in Kontakt kommen kann, wobei sich die Rillenflanken gegenüber der Vorderflanke beziehungsweise der folgenden Flanke befinden, um eine Klemmkraft zwischen der inneren Schraube und der äußeren Schraube zu erzeugen.

9. Kolbensatz nach einem der Ansprüche 1 bis 6, wobei jedes Gewinde (45A) der äußeren Schraube (45) an den Zwischenteilen der Vorderflanke (45G) und der folgenden Flanke (45H) desselben entsprechend mit Schulterabschnitten (45K, 45K) bereitgestellt wird und das Gewinde (45A) der äußeren Schraube und die entsprechende Rille (44A) der inneren Schraube in Profil und Abmessungen derart eingestellt sind, dass die Schulterabschnitte mit den Rillenflanken (44G, 44H) der Rille entsprechend in Kontakt kommen können, um eine Klemmkraft zwischen der inneren Schraube und der äußeren Schraube zu erzeugen.

10. Kolbensatz nach einem der Ansprüche 1 bis 9, wobei der Kolben (12) eine innere Schraube (14) aufweist, die mittels eines Gewindes in die Kolbenstange (13) eingreift, wobei die innere Schraube darin einen Zylinderraum (14B) definiert und Rillen (14A), die sich spiralförmig von dem Zylinderraum in Richtung des Außenumfangs des Kolbens erstrecken, wobei die Kolbenstange die äußeren Schraube (15) aufweist, die mittels eines Gewindes in den Kolben eingreift, und die äußeren Schraube einen Zylinder (15B) und Gewinde (15A) aufweist, die sich spiralförmig in radialer und nach außen gerichteter Richtung von dem Zylinder erstrecken.

11. Medizinische Spritze, **dadurch gekennzeichnet, dass** sie einen Spritzenzylinder und den Kolbensatz nach einem der Ansprüche 1 bis 10 umfasst, der verschiebbar in einer Form mit Gewindeeingriff in den Spritzenzylinder eingepasst ist.

## Revendications

1. Kit de poussoir en tant que composant pour une seringue à médicament pourvue d'un canon de seringue et utile pour injecter un médicament, ledit kit de poussoir comprenant un piston (12) ayant un diamètre de 13 mm ou plus petit et une hauteur de 10 mm ou plus petite, fait d'un élastomère et ayant une vis interne (14; 24 ; 34 ; 44 ; 54 ; 64), et une tige de poussoir (13) faite d'un matériau plus dur que le piston et ayant une vis externe (15; 25; 35; 45; 55; 65);
dans lequel la vis interne et la vis externe ont respectivement un profil de rainure de base et un profil de filet de base mutuellement non complémentaires ; et
un flanc suivant (15H ; 25H; 35H; 45H; 55H; 65H) de chaque filet (15A ; 25A ; 35A; 45A; 55A; 65A) de la vis externe et un flanc de rainure (14H ; 24H; 34H; 44H ; 54H; 64H) d'une rainure correspondante (14A; 24A; 34A; 44A; 54A; 64A) de la vis interne sont formés de telle sorte que lorsque le piston et la tige de poussoir sont mis en prise filetée l'un avec l'autre et installés de manière coulissante dans le canon de seringue et que le piston coulisse ensuite dans une direction de traction à l'aide de la tige de poussoir, le flanc suivant puisse venir en contact avec le flanc de rainure qui est situé face à face avec le flanc suivant plus fortement sur une première partie (15I; 65I) du flanc suivant que sur une deuxième partie (15J ; 65J) du flanc suivant et plus faiblement sur la deuxième partie que sur la première partie pour accroître la force de prise de filet entre la vis externe et la vis interne sans expansion sensible du piston par la vis externe ;
et dans lequel l'angle (α) du flanc suivant par rapport à la direction de traction du piston et l'angle (β) du flanc de rainure par rapport à la direction de poussée du piston sont chacun de 90 à 150°, et les angles sont différents l'un de l'autre.

2. Kit de poussoir selon la revendication 1, dans lequel l'un (15A; 25A; 34A; 44A; 55A) du profil de rainure de base de la vis interne et du profil de filet de base de la vis externe a une forme de filet de vis trapézoïdale, et l'autre (14A; 24A; 34A ; 44A; 54A) a l'une d'une forme de filet ronde, d'une forme de filet carrée et d'une autre forme de filetage trapézoïdale ayant des côtés obliques dont les inclinaisons sont différentes de celles de côtés obliques de la forme de filet de vis trapézoïdale mentionnée en premier.

3. Kit de poussoir selon la revendication 1, dans lequel l'un (15H; 25H; 44H; 64H) du flanc suivant et du flanc de rainure qui est situé face à face avec le flanc suivant a une surface plate, et l'autre (14H ; 24H; 45H; 65H) a une surface courbe.

4. Kit de poussoir selon la revendication 3, dans lequel la surface courbe est formée sur une partie au moins du flanc suivant (45H ; 65H).

5. Kit de poussoir selon la revendication 3 ou 4, dans lequel l'un (14A ; 24A ; 45A; 65A) du profil de rainure de base de la vis interne et du profil de filet de base de la vis externe a une forme de filet ronde, et l'autre (15A ; 25A ; 44A ; 64A) a une forme de filet de vis trapézoïdale ou une forme de filet carrée.

6. Kit de poussoir selon l'une quelconque des revendications 1 à 5, dans lequel un flanc menant (15G ; 25G; 35G; 45G; 55G; 65G) de chaque filet (15A; 25A ; 35A; 45A; 55A; 65A) de la vis externe (15 ; 25 ; 35 ; 45 ; 55 ; 65) et un flanc de rainure opposé (14G; 24G; 34G; 44G; 54G; 64G) de la rainure correspondante (14A; 24A ; 34A; 44A; 54A; 64A) de la vis interne (14 ; 24 ; 34 ; 44 ; 54; 64) sont formés de telle sorte que lorsque le piston (12) coulisse dans une direction de poussée à l'aide de la tige de piston (13) dans le canon de seringue, le flanc menant puisse venir en contact avec le flanc de rainure opposé qui est situé face à face avec le flanc menant plus fortement sur une troisième partie (15C ; 65C) du flanc menant que sur une quatrième partie (15D ; 65D) du flanc menant et plus faiblement sur la quatrième partie que sur la troisième partie pour accroître la force de prise de filet entre la vis externe et la vis interne sans expansion sensible du piston par la vis externe

7. Kit de poussoir selon l'une quelconque des revendications 1 à 6, dans lequel la vis externe (25) et la vis interne (24) sont déterminés en leur profil et leurs dimensions de telle sorte que chaque paire de filets adjacents (25A, 25A) de la vis externe puisse venir en contact, sur des parties côté base (25B, 25B) de ceux-ci, avec une partie de filet inter-rainure (28) de la vis interne, ladite partie de filet inter-rainure étant située entre les deux filets, avec la partie de filet inter-rainure serrée sur une partie d'extrémité libre de celle-ci par les parties côté base, pour produire une charge de serrage entre la vis interne et la vis externe.

8. Kit de poussoir selon l'une quelconque des revendications 1 à 6, dans lequel chaque filet (25A ; 35A) de la vis externe (25 ; 35) et sa rainure correspondante (24A; 34A) de la vis interne (24 ; 34) sont déterminés en leur profil et leurs dimensions de telle sorte que le filet puisse venir en contact, en des parties de coin d'extrémité (25F, 25F ; 35F, 35F) de celui-ci situées entre une crête de celui-ci et un flanc menant (25G ; 35G) et le flanc suivant (25H; 35H) de celui-ci, respectivement, avec des flancs de rainure (24G, 24H; 34G, 34H) de la rainure, lesdits flancs de rainure étant situés face à face avec le flanc menant et le flanc suivant, respectivement, pour produire une charge de serrage entre la vis interne et la vis externe.

9. Kit de poussoir selon l'une quelconque des revendications 1 à 6, dans lequel chaque filet (45A) de la vis externe (45) est pourvu, sur des parties intermédiaires d'un flanc menant (45G) et du flanc suivant (45H) de celui-ci, de parties d'épaulement (45K, 45K), respectivement, et le filet (45A) de la vis externe et la rainure correspondante (44A) de la vis interne sont déterminés en leur profil et leurs dimensions de telle sorte que les parties d'épaulement puissent venir en contact avec les flancs de rainure (44G, 44H) de la rainure, respectivement, pour produire une charge de serrage entre la vis interne et la vis externe.

10. Kit de poussoir selon l'une quelconque des revendications 1 à 9, dans lequel le piston (12) a la vis interne (14) apte à venir en prise filetée avec la tige de poussoir (13), la vis interne définit dans celui-ci un espace cylindrique (14B) et des rainures (14A) s'étendant en hélice depuis l'espace cylindrique vers une circonférence extérieure du piston, la tige de poussoir a la vis externe (15) apte à venir en prise filetée avec le piston, et la vis externe a un cylindre (15B) et des filets (15A) s'étendant en hélice dans une direction radiale et vers l'extérieur depuis le cylindre.

11. Seringue à médicament **caractérisée en ce qu'**elle comprend un canon de seringue et le kit de poussoir selon l'une quelconque des revendications 1 à 10 inséré de manière coulissante dans le canon de seringue sous une forme en prise filetée.
